# EUROPEAN PATENT APPLICATION

(11) **EP 0 848 953 A1**
(43) Date of publication of application: **24.06.1998**
(21) Application number: 97122232.8
(22) Date of filing: 17.12.1997
(51) Int. Cl.: A61K 31/34

(54) **5-Hydroxy- and 5-acyloxy-2,3-dihydro-4,6,7-trimethyl-2-benzofurane-acetic acids and their esters for treating and preventing diabetic complications**

(30) Priority: 20.12.1996 IT MI962701
(71) Applicant: Biomedica Foscama Industria Chimico-Farmaceutica S.p.A., 03013 Ferentino (IT)
(72) Inventor: Dorigotti, Luciano, 20080 Basiglio (MI) (IT); Ceccarelli, Stefano, 03100 Frosinone (FR) (IT); Gritti, Franco c/o Biomedica Foscama SpA, 03013 Ferentino (FR) (IT)
(74) Representative: Savi, Alberto

(57) **Abstract**

It is disclosed to the use of 5-hydroxy- and 5-acyloxy-2,3-dihydro-4,6,7-trimethyl-2-benzofurane-acetic acids and their esters and of pharmaceutical formulations containing them as active ingredients in a therapy for treating and preventing chronic diabetic complications.

## Description

This invention refers to the use of 5-hydroxy- and 5-acyloxy-2,3-dihydro-4,6,7-trimethyl-2-benzofurane-acetic acids and their esters and of pharmaceutical formulations containing them as active ingredients in a therapy for treating and presenting chronic diabetic complications.

It is known that a diabetic patient encounters a number of complications causing a high degree of illness and mortality. The symptoms appear on the average 15 to 20 years after the onset of hyperglycemia, and may even show up simultaneously. The most common chronic complications of insuline-dependent diabetes mellitus and non-insuline dependent diabetes mellitus are microangiopathies (nefropathy, retinopathy), macroangiopathies (aterosclerosis, ischemic myocardium illness, cerebrovascular pathologies and peripheral arteriopathies), neuropathies, erectile disfunctions and male impotence, autonomic neuropathies (constipation, gastroparesia, urinary bladder disfunctions), lipoides diseases, and hypertension. The causes of such complications are basically attributable to vascular disfunctions (especially in small vessels), correlated with damages of the endotelium and consequent alterations of the functions dependent on the same.

There are to this date, especially for the non-insulin dependent diabetes, no long-term studies capable of demonstrating any effectiveness of medical action based on the oral administration of drugs or insulin in retarding or preventing such complications (B.H.R. Wolffenbuttel & T.W. van Haeften, Drugs 1995, 50, 263-288).

From a biochemical viewpoint, it is generally acknowledged that the alteration of the "poliol pathway" on one side, and the non-enzymatic glycation processes of the plasmatic proteins and the vascular wall proteins on the the other side play an important role in causing vascular disfunctions in diabetic patients. These processes are, by the formation of terminal glycation byproducts (AGE) probably at the root, among other things, of the alteration of the control mechanisms of the vascular tone, which is in turn caused by a reduced NO-activity. The diabetic patients, especially those of a non-insulin dependent kind, in fact exhibit an insufficient vasodilatation action in the presence of an endotelial disfunction (see in this regard: J. Goodfellow et al., Brit. Med.J. 1996, 312, 744-745.

The US 4,999,350 document describes the 5-hydroxy- and 5-aciloxy-2,3-dihydro-4,6,7-trimethyl-2-benzofurane-acetic acids and their esters, defined by the general formula (I)

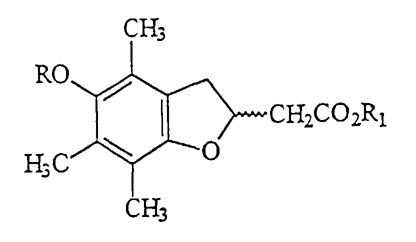

In which R is hydrogen, C₁ - C₇ acyl, C₂ - C₆ carboxylic acid hemiacyl; R₁ is hydrogen, C₁ - C₂₀ alkyl; alkyl-ether of the type -CHR₂ -(CH₂)ₙ-0-(CH₂)ₘ in which R₂ is a lower alkyl C₂ - C₄, and n and m vary from 0 to 6, alkyl diether of the type -(CH₂)ₙ -0-(CH₂)ₘ-0-(CH₂)ₚ -CH₃ in which n and m vary from 1 to 6 and p varies from 0 to 6, alkylamine of the type -CHR₂ -(CH₂ ) ₙ-NR₃R₄ in which n varies from 0 to 6, R₂ has the meaning outlined above, and R₃ and R₄ are C₂ - C₄ lower alkyls, N-alkylhetero- cycle of the type 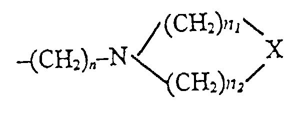 in which X is CH₂, S, 0, NR₂ (where R₂ has the former meaning), n varies from 1 to 6, n₁ and n₂ vary from 1 to 3; and their pharmaceutically acceptable salts. These compounds, and in particular the (±) -5-acetoxy-2,3-dihydro-4,6,7-trimethyl-2-benzofurane-acetic acid (raxofelast) (INN) and its active metabolyte (±) -2,3-dihydro-5-hydroxy-4,6,7-trimethyl-2-benzofurane-acetic acid are endowed with antioxidant and lipidic peroxidation inhibiting properties , as described by A. Bindoli et al. In Pharmacol, Res. 1991, 24, 369-375, and have also evidenced mucoregulating and anti-ischemic action (see for ex.: G.M. Campo et al., Res. Commun. Chem. Pathol. Pharmacol. 1992, 76, 287-303).

By this invention it was surprisingly discovered that substances having the formulation (I) as described above are capable of reestablishing the endotelium-dependent vasodilatation reaction compromised in the diabetic patients. They may therefore be used in a therapy for treating and preventing the chronic complications of the non-insulin-dependent and insulin-dependent diabetes mellitus with a micro- and/or macrovascular component.

This invention therefore refers to the use of a compound of the formulation (I), as defined above as an active principle for preparing drugs suitable for preventing and treating all chronic complications of diabetes mellitus with a vascular component, such as the microangiopathies (nefropathy, retinopathy), the macroangiopathies (aterosclerosis; ischemic miocardium illness, cerebrovascular and peripheral arteriopathies), the neuropathies, the erectile disfunctions and male impotence, the autonomic neuropathies (constipation, gastroparesia, disfunctions of the urinary bladder), the lipoides diseases, and hypertension.

A particularly preferred use is that of a compound of the formulation (I), in which R is acetyl and R₁ is hydrogen (raxofelast) (INN), for the same purposes as outlined abode. The compounds of the formula (I) according to this invention can be prepared by the methods reported in the literature for the 2,3-dihydro-5-benzofuranolic derivatives and in particular as described in US-patent 5,041,568. The formula (I) compound in which R is acetyl and R₁ is hydrogen (raxofelast) (INN) can for instance be prepared by cycling a suitable derivative of the 4-(2,5-dihydroxy-3,4,6-trimethylphenyl)-(E)-2-butenoic acid in an aqueous alcaline medium, followed by 0-acetylation of the resulting (±) -2,3-dihydro-5-hydroxy-4,6,7-trimethyl-2-benzofurane-acetic acid.

For all the therapeutic uses of this invention the formula (I) compounds may be administered orally, parenterally, transdermally or transmucoidally, rectally or intrauretrally or by inhalation in formulations containing them as active ingredients, at a therapeutically effective dosage along with conventional, non-toxic vehicles. The term parenteral used here includes subcutaneous, intramuscular, endovenous and intracavernous injections.

To prepare drugs useful for a therapy, these compounds may eventually be used individually or in a mixture between them. The pharmaceutical compositions for oral application comprise pills and capsules in which the active ingredient is admixed with non-toxic and pharmaceutically acceptable vehicles. These vehicles may be inert diluents, such as calcium carbonate, sodium carbonate, lactose, calcium phosphate or sodium phosphate; granulating or dispersing agents such as corn starch or alginic acid; binders such as starch or gelatine; lubricating agents such as magnesium stearate, stearic acid or talcum.

The pills may be non-coated or coated by conventional techniques by a skilled man in the art, so as to delay their disintegration and absorption in the gastrointestinal tract and achieve a delayed and time-persistant action. The aqueous suspensions generally contain the active ingredients in a mixture with appropriate vehicles. The vehicles may be suspending agents such as sodium carboxymethylcellulose, hydroxypropylmethylcellulose, sodium alginate, polpynilpyrrolidone; dispersing and wetting agents. They may also contain one or more preserving agents, such as ethyl- or n-propyl-p-hydroxybenzoate; one or more aromatising agents; and one or more sweetening agents.

The oily suspensions may be formulated by dispersing the active ingredient in a vegetable or mineral oil; they may contain aromatising or sweetening agents to render the preparation palatable. The powders and dispersible granules suitable for preparing an aqueous suspension by the addition of water contain the active principle in a mixture with a dispersing or wetting agent, a suspending agent and one or more preserving agents.

The pharmaceutical compositions suitable for the application of this invention may also be in an oil/water emulsified form. The oily phase may be constituted by a vegetable or mineral oil. The emulsifying agents may be natural gums such as from the acacia tree, or natural phosphatides, for example lecitine or natural or synthetic fatty acids. The syrups and elisirs can be formulated as sweetening agents, for example glycerol, sorbitol or sucrose.

The pharmaceutical compositions may also be in the form of sterile aqueous or oily injectable solutions or suspensions, formulated by known methods by using non-toxic solvents or diluents suitable for parentheral use.

The compounds of this invention may also be administered rectally in the form of suppositories. These compositions may be prepared by mixing the active ingredient with a suitable non-irritating vehicle which is solid at room temperature, but liquid at rectal temperature. The polyethyleneglycols and cocoa butter are suitable for this purpose.

The therapeutically or prophylactically effective quantity of a formula (I) compound for the application of this invention will depend on a number of factors, among which, for instance, the patient's body weight and age, the gravity of the specific pathology subject to treatment, and the method of administration. For each individual administration the quantity of the formula (I) compound generally applicable may vary from 5 - 1500 mg/die. However, an effective quantity of the formula(I) compound for treating diabetic complications such as for instance a nephropathy will generally be in the range of 0.1 - 75 mg/kg body weight per day, and more frequently in the range of 1-25 mg/kg body weight per day.

The use of compounds of a general formula (I) according to this invention is illustrated in the following example, but is in no way limited to the same:

### Example:

Measurements were undertaken on the upper limbs of no. 10 subjects affected by non-in sulin-dependent diabetes mellitus which had received gradually increasing dosages of an acetylcolinomimetic agent (metacoline) by intraarterial means, both under base conditions and after repeated treatment (5/6 days) with 1200 mg/die of raxofelast (INN) by oral means. The flow increases in the vascular region of the brachial artery were compared with those of no. 10 healthy (control) subjects subjected to the same dosages of metacoline.

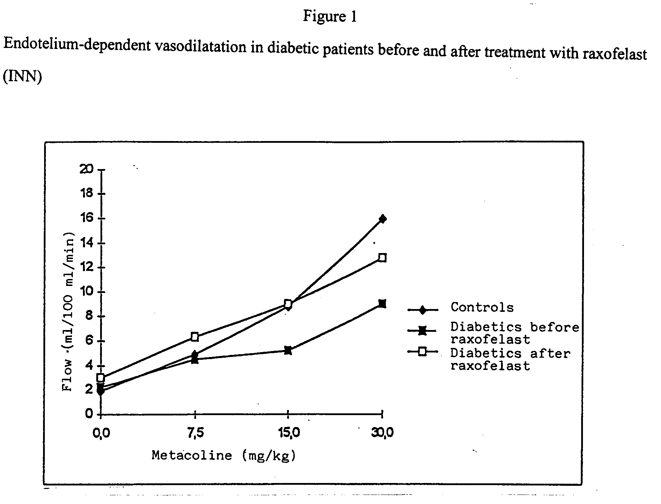

Figure 1 shows the evolution of the vasodilatation reaction (arterial flow measured by pletismograph) as a function of the pharmaceutical stimulant (metacoline) for the group of diabetic patients before and after the treatment with raxofelast (INN) and for the control group. The resulting pletismographic curves indicate that:
a) The vascular reaction of the diabetic patients to the administration of the colinergic agonist is significantly less than that observed in normal subjects, thus indicating a compromission of the endotelium-dependent vasodilatation (VED);
b) In the subjects suffering from non-insuline-dependent diabetes the VED appears to be significantly improved after administering raxofelast (INN).

## Claims

1. A use of the compound of formula (I) 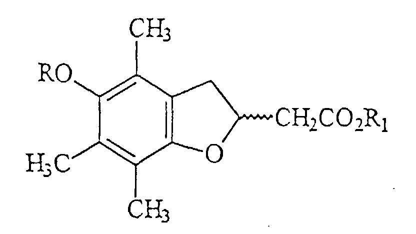 in which R is hydrogen, C₁ - C₇ acyl, C₂ - C₆ bicarboxylic acid hemiacyl; R₁ is hydrogen, C₁ -C₂₀ alkyl: alkyl-ether of the type -CHR₂ -(CH₂)ₙ-0-(CH₂)m in which R₂ is a C₂ - C₄ lower alkyl, and n and m vary from 0 to 6, alkyl diether of the type -(CH₂)ₙ -0-(CH₂)ₘ-0(CH₂)ₚ -CH₃ in which n and m vary from 1 to 6 and p varies from 0 to 6, alkylamine of the type -CHR₂ -(CH₂ ) ₙ -NR₃R₄ in which n varies from 0 to 6, R₂ has the meaning described above, and R₃ and R₄ are C₂ - C₄ lower alkyl, N-alkylheterocycle of the type 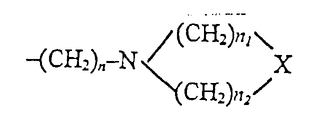 in which X is CH₂, S, 0, NR₂ (where R₂ has the former meaning), n varies from 1 to 6, n₁ and n₂ vary from 1 to 3; and their pharmaceutically acceptable salts, for preparing a drug useful in a therapy for treating and preventing chronic diabetes complications.

2. A use of the formula (I) compound according to the claim 1, in which R is acetyl and R₁ is hydrogen.

3. A use of the formula (I) compound according to the claims 1 or 2 for treating and preventing diabetic nephropathies.

4. A use of the formula (I) compound according to the claims 1 or 2 for treating and preventing diabetic retinopathies.

5. A use of the formula (I) compound according to the claims 1 or 2 for treating and preventing diabetic macroangiopathies.

6. A use of the formula (I) compound according to the claims 1 or 2 for treating and preventing diabetic neuropathies.

7. A use of the formula (I) compound according to the claims 1 or 2 for treating and preventing erectile disfunctions and male impotence in diabetic patients.

8. A use of the formula (I) compound according to the claims 1 or 2 for treating and preventing autonomic diabetic neuropathies.

9. A use of the formula (I) compound according to the claims 1 or 2 for treating and preventing lipoides disfunctions in diabetic patients.

10. A use of the formula (I) compound according to the claims 1 or 2 for treating and preventing hypertension in diabetic patients.

11. A use of the formula (I) compound according to one of the claims from 1 to 10 for preparing a drug containing 5-1500 mg/die of active ingredient.
